# EUROPEAN PATENT SPECIFICATION

(11) **EP 1 034 001 B1**
(45) Date of publication and mention of the grant of the patent: **04.08.2004**
(21) Application number: 98931391.1
(22) Date of filing: 19.06.1998
(51) Int. Cl.: A61K 39/395, A61K 38/00

(54) **CD154 BLOCKADE THERAPY FOR THERAPEUTIC PROTEIN INHIBITOR SYNDROME**
CD154-BLOCKADETHERAPIE FÜR DAS SYNDROM DER HEMMUNG DER THERAPEUTISCHEN PROTEINE
THERAPIE PAR BLOCAGE DES CELLULES CD154 CONTRE LE SYNDROME INHIBITEUR DE PROTEINES THERAPEUTIQUES

(30) Priority: 20.06.1997 US 50276 P
(43) Date of publication of application: 13.09.2000
(73) Proprietor: Biogen Idec MA Inc., Cambridge, MA 02142 (US)
(72) Inventor: ADELMAN, Burt, Concord, MA 01742 (US)
(74) Representative: von Menges, Albrecht
(86) International application number: PCT/US1998/012773
(87) International publication number: WO 1998/058672

(56) References cited:
- WO-A-98/30241
- US-A- 5 474 771
- FOY T M ET AL: "In vivo CD40-gp39 interactions are essential for thymus-dependent humoral immunity. II. Prolonged suppression of the humoral immune response by an antibody to the ligand for CD40, gp39." JOURNAL OF EXPERIMENTAL MEDICINE, (1993 NOV 1) 178 (5) 1567-75. JOURNAL CODE: I2V. ISSN: 0022-1007., XP000647639 United States cited in the application
- MOHAN C ET AL: "Interaction between CD40 and its ligand gp39 in the development of murine lupus nephritis." JOURNAL OF IMMUNOLOGY 154 (3). 1995. 1470-1480. ISSN: 0022-1767, XP002062342 cited in the application
- DURIE, FIONA H. ET AL: "Prevention of collagen - induced arthritis with an antibody to gp39, the ligand for CD40" SCIENCE (WASHINGTON, D. C., 1883-) (1993), 261(5126), 1328-30 CODEN: SCIEAS;ISSN: 0036-8075, XP002064747 cited in the application
- NOELLE, RANDOLPH J.: "The role of gp39 ( CD40L ) in immunity" CLIN. IMMUNOL. IMMUNOPATHOL. (1995), 76(3, PT. 2), S203-S207 CODEN: CLIIAT;ISSN: 0090-1229, XP002080010
- BUHLMANN J E ET AL: "Therapeutic potential for blockade of the CD40 ligand, gp39." JOURNAL OF CLINICAL IMMUNOLOGY, (1996 MAR) 16 (2) 83-9. REF: 56 JOURNAL CODE: HRC. ISSN: 0271-9142., XP002079820 United States
- Qian et al: Eur. J. Immunol. 30: 2548-2554, 2000.
- Bi et al: Nature Genetics 10:119-121, 1995.
- Fang-An Chen et al: The Journal of Immunology, vol. 155, 1995, 2833-2840

## Description

### Field of the Invention

The invention relates generally to the suppression of unwanted immune responses, particularly of counter-adaptive T-lymphocyte mediated immune responses. The invention relates in particular to the prevention, treatment, suppression and reversal of immunological inhibition of the therapeutic activity of exogenously-administered proteins or other biological therapeutic agents.

### Background of the Invention

Hemophilia A is an X-linked genetic deficiency disease that affects one to two males in every 10,000 live births. Individuals with hemophilia A have a partial or complete functional deficiency of endogenous clotting Factor VIII (FVIII), and must receive purified or recombinant FVIII replacement therapy. Lusher et al. (1993), 328 N. Engl. J. Med. 453-459. Approximately 15% of individuals with hemophilia A develop high-titer antibody responses (i.e., > 10 BU/mL) to their FVIII replacement therapeutic. These individuals are referred to as "high responders." McMillan et al. (1988), 71 Blood 344-348. Such antibodies, called FVIII inhibitors, block the function (bioactivity) of the replacement FVIII therapeutic agent, by binding to the administered, exogenous FVIII. This counter-adaptive, humoral immune response makes the treatment of bleeding events in high responders problematic. Minor bleeding episodes (e.g., hemarthroses) are often successfully treated with activated prothrombin complex concentrates (e.g., Autoplex or FEIBA, FVIII inhibitor bypass activity), but severe bleeding is difficult to control with these agents. Brettler (1996), 9 Clin. Hematol. 319-329. The majority of high responders have low levels of the FVIII inhibitor antibodies until they receive a subsequent infusion of FVIII, which stimulates (boosts) the production of the blocking antibodies. Therefore, the re-induction of inhibitory antibodies in high responder individuals is highly predictable. Brettler (1996), 9 Clin. Hematol. 319-329. Prophylaxis with FVIII has been shown to reduce the incidence of intraarticular hemorrhage and chronic arthropathy in hemophiliacs. Liesner et al. (1996), 92 Br. J. Haematol. 973-978. However, because of the FVIII specific humoral immune response in high responders, such individuals cannot receive prophylactic therapy with FVIII.

High responders with an inducible, vigorous immune response to FVIII are often treated with regular infusions of very high doses of FVIII in regimens designed to induce "tolerance" to the exogenous FVIII therapeutic. Brettler (1996), 9 Clin. Hematol. 319-329. In some cases, immunomodulator agents (glucocorticoids, cyclophosphamide, intravenous immunoglobulin (IVIg)) are added to the tolerance regimens. Tolerance therapy is effective in 50% to 80% of high responder individuals. Mariani et al. (1994), 72 Thromb. Haemostasis 155-158. The cost of such therapy ranges from $200,000US to nearly $1 million US per individual. Because infusion of FVIII induces very high levels of inhibitors in high responders, during the tolerance induction period - which often lasts from three to eight months, these individuals cannot be treated with FVIII if they do have a bleed. Brettler (1996), 9 Clin. Hematol. 319-329.

Following successful tolerance induction, individuals are often maintained on prophylactic FVIII infusions twice to three times weekly. Brettler (1996), 9 Clin. Hematol. 319-329. The mechanism of tolerance induction using these protocols is unclear, but may involve the induction of anti-idiotypic antibodies (Gilles et al. (1996), 97 J. Clin. Invest. 1382-1388) or more direct suppression of the B cell clones making the FVIII inhibitor antibodies. In addition, IVIg preparations contain anti-idiotypic antibodies to FVIII inhibitors, which may explain the efficacy of this therapy in some high responders. Sultan et al. (1991), 91 Am. J. Med. 5A-35S-5A-39S. In very severe and life-threatening cases that necessitate the use of FVIII therapy for bleeding, FVIII inhibitors can be removed by extracorporeal immunoabsorption on anti-Ig or Protein A columns. Knobl et al. (1995), 74 Thromb. Haemostasis 1035-1038; Gjorstrup et al. (1991), 61 Vox Sang 244-250. These protocols are time-consuming and result in 50% to 75% reduction in total serum immunoglobulin (Ig) levels (Knobl et al. (1995),74 Thromb. Haemostasis 1035-1038) thus potentially increasing the risk of infection.

Similar complications of protein replacement therapy have been encountered in treatment of other congenital or acquired protein deficiency diseases, including deficiencies of other clotting factors, blood or plasma proteins, growth factors, and the like. Furthermore, analogous complications have been encountered in other clinical settings, such as where a recombinantly produced counterpart of an endogenous, but rare or sequestered protein is administered therapeutically. For example, analogous complications have been encountered in therapies involving the administration of recombinant cytokines, lymphokines, growth factors or enzymes. One example is the administration of erythropoeitin (EPO) for treatment of anemia. Another is the administration of interferon β (IFN β) for treatment of multiple sclerosis (MS). Still another is the administration of human growth hormone (hGH) for treatment to accellerate growth. Analogous complications also have been encountered where a microbial protein is administered therapeutically, such as where streptokinase is administered for treatment of stroke or another type of vascular occlusion.

There is accordingly a need for improved or more effective immunosuppressive or immunomodulatory treatments for minimizing or suppressing the development of inhibitory antibodies that bind to, and block the therapeutic activity of, exogenously administered proteins. In particular, there is a need for treatments that do not require pan-T cell immunosuppression, i.e., treatments that do not leave the recipient vulnerable to malignancies or opportunistic infection. More pointedly, there is a need for reversing or suppressing inhibitor syndromes that preclude the administration of a needed protein therapeutic, such as FVIII, to individuals in need thereof.

### Summary of the Invention

It is an object of this invention to provide an immunomodulatory agent that mitigates counter-adaptive T cell responses without the need for pan-T cell immunosuppression. Another object is to provide an immunomodulatory agent that mitigates seventy of a counter-adaptive humoral immune response to a needed, exogenous protein therapeutic. Another object is to provide an immunomodulatory agent that delays onset of a counter-adaptive humoral immune response to a needed, exogenous protein therapeutic. Another object is to provide an immunomodulatory agent that suppresses or reverses a counter-adaptive humoral immune response to a needed, exogenous protein therapeutic. A further object is to provide an immunomodulatory agent that interrupts delivery of a costimulatory signal to activated T cells, particularly a costimulatory signal for immunoglobulin production. A particular object is to provide a CD40:CD154 binding interruptor, such as a CD154 blocking agent, for use in therapy, particularly for use in therapy to mitigate, delay onset of, or reverse a counter-adaptive inhibitory antibody response to a needed, exogenous protein therapeutic agent, such as FVIII.

The present invention rests on the discovery that use of a CD40:CD154 binding interruptor, such as a CD154 blocking agent, attenuates, mitigates, suppresses, prevents, delays, inhibits or reverses counter-adaptive inhibitory antibody responses to protein antigens, *without* the need for pan-suppression of the recipient's immune system. More precisely, the present invention rests on the discovery that use of a CD154 blocking agent attenuates, mitigates, suppresses, prevents, delays, inhibits or reverses undesirable inhibitory humoral immunity that blocks bioactivity of a protein therapeutic administered to an individual to replace or augment native bioactivity of an endogenous, but defective protein, such as a clotting factor, e.g., FVIII.

The invention accordingly relates to the use of the CD40:CD145 binding interruptor being a monoclonal antibody having the antigen-specific binding characteristics of the 5c8 antibody produced by ATCC Accession No. HB 10916, as disclosed in US 5474711, for the manufacture of a medicament for attenuating the severity of exogenous protein inhibitor syndrome in hemophiliac humans receiving coagulation Factor VIII or Factor IX as an exogenous proteins.

The foregoing and other objects, features and advantages of the present invention, as well as the invention itself, will be more fully understood from the following description of preferred embodiments.

### Detailed Description of the Invention

T cell activation, and immunological processes dependent thereon, requires both T cell receptor (TCR) mediated signals and simultaneously delivered costimulatory signals. An important costimulatory signal is delivered by the ligation of CD40 on an antigen-presenting cell, such as a B cell, by CD40L (CD154) on a T cell. Human CD40 is a 50 kD cell surface protein expressed on mature B cells, as well as on macrophages and activated endothelial cells. CD40 belongs to a class of receptors involved in programmed cell death, including Fas/CD95 and the tumor necrosis factor (TNF) alpha receptor. Human CD154 (CD40L) is a 32 kD type II membrane glycoprotein with homology to TNF alpha that is transiently expressed, transiently, primarily on activated T cells. CD40:CD154 binding has been shown to be required for all T cell-dependent antibody responses. In particular, CD40:CD 154 binding provides anti-apoptotic and/or lymphokine stimulatory signals.

The importance of CD40:CD154 binding in promoting T cell dependent biological responses was more fully appreciated when it was discovered that X-linked hyper-IgM syndrome (X-HIGM) in humans is the phenotype resulting from genetic lack of functional CD154. Affected individuals have normal or high IgM levels, but fail to produce IgG, IgA or IgE antibodies, and suffer from recurrent, sometimes severe, bacterial and parasitic infections, as well as an increased incidence of lymphomas and abdominal cancers. A similar phenotype is observed in non-human animals rendered nullizygous for the gene encoding CD154 (knockout animals). B cells of CD154 nullizygotes can produce IgM in the absence of CD40L:CD154 binding, but are unable to undergo isotype switching, or to survive normally after affinity maturation. Histologically, lymph node germinal centers fail to develop properly, and memory B cells are absent or poorly developed. Functionally, these defects contribute to a severe reduction or absence of a secondary (mature) antibody response. Defects in cellular immunity are also observed, manifested by an increased incidence of bacterial and parasitic infections. Many of these cell-mediated defects are reversible by administration of IL-12 or IFN-gamma. These observations substantiate the view that normal CD40:CD154 binding promotes the development of Type I T-helper cell immunological responses.

Blockade of the CD40:CD154 interaction during immunization with protein antigens can specifically block the antibody response to that antigen in mice. Foy et al. (1993), 178 J. Exp. Med. 1567-1575. For example, anti-CD154 antibodies can block the induction of anti-collagen antibodies in collagen-induced arthritis. Durie et al. (1993), 261 Science 1328-1330. Anti-CD154 antibodies can reduce anti-dsDNA and anti-nucleosomal autoantibodies in mice with spontaneous lupus. Mohan et al. (1995), 154 J. Immunol. 1470-1480. In addition, anti-CD154 antibodies can reduce symptoms in mice with experimental autoimmune encephalomyelitis (EAE), a model of MS. Similar results have been reported in rodent models of graft-versus-host-disease, mercuric chloride induced glomerulonephritis, and inflammatory bowel disease.

CD40:CD 154 blockade thus may provide potentially powerful therapies for attenuating or ameliorating unwanted humoral immune responses, particularly in the context of autoimmune diseases or, indeed, wherever the target antigen is a protein of therapeutic value, which value is impeded by a counter-adaptive immune response. However, despite numerous reports of promising results, studies performed in rodent models of induced counter-adaptive immunological disease (e.g., autoimmunity) have correlated poorly with the outcome of testing in actual disease contexts, or even in larger animal preclinical model systems (e.g., primates).

Disclosed herein are protocols for assessing the effects of a preferred CD154 blocking agent, a humanized MAb having the antigen-specific binding properties of MAb 5c8 (Lederman et al., J. Exp. Med. 175:1091-1101,1992), in preclinical models believed predictive of therapeutic efficacy in treatment of exogenous protein inhibitor syndromes. Specifically, the present models involve CD 154 blockade therapy to attenuate or ameliorate bioinhibitory humoral immunity specific for clotting factors (e.g., FVIII) and lymphokines (e.g., IFN β). These models can be adapted, through no more than routine manipulation, for use to establish efficacy of CD 154 blockade therapy to attenuate or ameliorate inhibitory humoral immunity directed against any protein of therapeutic value.

The following discussion illustrates and exemplifies the variety of contexts and circumstances in which the invention can be practiced, as well as providing proof-of-principle studies involving specific embodiments of the invention.

### Subjects for Treatment

The invention can be used for treatment or prophylaxis of any mammalian subject in need of, or already receiving, protein replacement therapy, indeed any protein therapeutic. Subjects accordingly are afflicted with, or at risk of, developing exogenous protein inhibitor syndrome. For example, hemophiliacs being treated with exogenous FVIII are at substantial risk of becoming "high responders," whereafter FVIII loses effectiveness for its intended purpose of suppressing bleeding events. Accordingly, the invention is particularly suitable for use with hemophiliacs. Procedures for determining whether a hemophiliac has developed an inhibitory response against therapeutically administered FVIII, and/or has become a high responder, are well known. See, e.g., Hematology: Clinical and Laboratory Practice, vol. 2, Bick, ed., Mosby-Year Book, Inc., publ. (1993), pp.1544-1548. Preferably, the subject mammal is a primate, more preferably a higher primate, most preferably a human. In other embodiments, the subject may be another mammal afflicted with, or at risk of, developing an exogenous protein inhibitor syndrome, particularly a mammal of commercial importance, or a companion animal or other animal of value, such as a member of an endangered species. Thus, subjects also include, but are not limited to, sheep, horses, cattle, goats, pigs, dogs, cats, rabbits, guinea pigs, hamsters, gerbils, rats and mice.

### Exemplary CD40:CD154 Binding Interruptors

Therapeutic compounds useful for practice of the invention include any compound that blocks the interaction of cell surface CD40 (e.g., on B cells) with CD40L (CD154) expressed, e.g., on the surface of activated T cells. CD40:CD154 binding interruptor compounds, such as CD154 blocking agents, that are specifically contemplated include polyclonal antibodies and monoclonal antibodies (MAbs), as well as antibody derivatives such as chimeric molecules, humanized molecules, molecules with reduced effector functions, bispecific molecules, and conjugates of antibodies. In a preferred embodiment, the antibody has substantially the same antigen-specific binding characteristics as MAb 5c8, as described in U.S. Patent 5,474,771. In a currently highly preferred embodiment, the antibody is a humanized 5c8 (hu5c8). Other known antibodies against CD154 include antibodies ImxM90, ImxM91 and ImxM92 (disclosed by Immunex Corp., Seattle WA), an anti-CD40L MAb commercially available from Ancell (clone 24-31, catalog # 353-020, Bayport, MN), and an anti-CD 154 MAb commercially available from Genzyme (Cambridge, MA, catalog # 80-3703-01). Also commercially available is an anti-CD154 MAb from PharMingen (San Diego, catalog #33580D). Numerous additional anti-CD154 antibodies have been produced and characterized (see, e.g., WO 96/23071 of Bristol-Myers Squibb.

The invention also includes use of CD 154 blocking agents that are derived from, or engineered from the above-mentioned and equivalent MAbs, such as complete Fab fragments, F(ab')₂ compounds, V_{H} regions, F_{V} regions, single chain antibodies (see, e.g., WO 96/23071), polypeptides, fusion constructs of polypeptides, fusions of CD40 (such as CD40Ig, as in Hollenbaugh et al., J. Immunol. Meth. 188:1-7, 1995, which is hereby incorporated by reference), and small molecule compounds such as small semi-peptidic compounds or non-peptide compounds, all capable of blocking or interrupting CD40:CD154 binding. Procedures for designing, screening and optimizing small molecules are provided in PCT/US96/10664, filed June 21, 1996 (WO 97/00895).

Thus, the invention can be practiced with MAb-derived, CD 154 blocking agents created using standard recombinant DNA techniques (Winter and Milstein, Nature 349: 293-99, 1991). One class of such CD154 blocking agents includes chimeric antibodies, or fusion proteins constructed by joining nucleic acid encoding the antigen binding domain of a non-human mammalian antibody (e.g., a mouse or rat antibody) of desired specificity to nucleic acid encoding a human immunoglobulin (Ig) constant region. Cabilly et al., United States Pat. No. 4,816,567; Morrison et al., Proc. Natl. Acad. Sci. 81: 6851-55, 1984. Chimeric antibody polypeptides expressed from such constructs generally have lower immunogenicity, when used for human therapy or prophylaxis, than the non-human antibody from which the chimera was derived. A second class of such CD154 blocking agents includes recombinant "humanized" or "primatized"antibodies. Humanized or primatized antibodies are antibodies are genetically engineered from non-human mammalian antibodies having the desired specificity, by replacing some or all of the codons for amino acids not required for antigen binding with codons for amino acids from corresponding regions of a human or primate Ig light or heavy chain gene. That is, they are chimeras comprising mostly human immunoglobulin sequences into which the regions responsible for antigen specific binding have been genetically inserted (see, e.g., PCT patent application WO 94/04679). Humanized antibodies generally have even lower immunogenicity in vivo than chimeric antibodies. Currently, a humanized MAb having substantially the same antigen specificity as MAb 5c8 (herein, hu5c8) is preferred for practice of the invention.

Another class of MAb-derived CD 154 blocking agents useful in the invention includes human antibodies, which can be produced in transgenic nonhuman mammals, into whom one or more human immunoglobulin transgenes have been integrated. Such animals may be used as a source for splenocytes for producing human hybridomas, as described in U.S. 5,569,825.

Of course, any antigen-specific binding fragment of one of the foregoing MAbs or MAb derived therapeutic agent can be used in the present invention, provided that the fragment is sufficiently large to sterically impede CD154 binding to its counter-receptor. Thus, MAb fragments and univalent MAbs can be used. Univalent antibodies comprise a heavy chain/light chain dimer bound to the Fc (or stem) region of a second heavy chain. "Fab region" refers to those portions of the chains which are roughly equivalent, or analogous, to the sequences which comprise the Y branch portions of the heavy chain and to the light chain in its entirety, and which collectively (in aggregates) have been shown to exhibit antibody activity. A Fab protein includes aggregates of one heavy and one light chain (commonly known as Fab'), as well as tetramers which correspond to the two branch segments of the antibody Y, (commonly known as F(ab)₂), whether any of the above are covalently or non-covalently aggregated, so long as the aggregation is capable of selectively reacting with a particular antigen or antigen family.

In addition, standard recombinant DNA techniques can be used to alter the binding affinities of recombinant antibodies with their antigens by altering amino acid residues in the vicinity of the antigen binding sites. The antigen binding affinity of a humanized antibody may be increased by mutagenesis based on molecular modeling (Queen et al., Proc. Natl. Acad. Sci. 86:10029-33, 1989; PCT patent application WO 94/04679). It may be desirable to increase or to decrease the affinity of the antibodies for CD 154, depending on the targeted tissue type or the particular treatment schedule envisioned. This may be done utilizing phage display technology (see, e.g., Winter et al., Ann. Rev. Immunol. 12:433-455, 1994; and Schier et al., J. Mol. Biol. 255:28-43, 1996. For example, it may be advantageous to treat a patient with constant levels of antibodies with reduced affinity for CD 154 for semi-prophylactic treatments. Likewise, antibodies with increased affinity for CD154 may be advantageous for short-term treatments.

### Routes of Administration

The CD40:CD154 binding interruptors, including CD 154 blocking agents, used in the invention can be administered in any manner which is medically acceptable. Depending on the specific circumstances, local or systemic administration may be desirable. Preferably, the agent is administered via a parenteral route such as by an intravenous, intraarterial, subcutaneous, intramuscular, intraorbital, intraventricular, intraperitoneal, subcapsular, intracranial, intraspinal, or intranasal injection, infusion or inhalation. The agent also can be administered by implantation of an infusion pump, or a biocompatible or bioerodable sustained release implant, into the recipient host, either before or after implantation of donor tissue. Alternatively, certain compounds of the invention, or formulations thereof, may be appropriate for oral or enteral administration. Still other compounds of the invention will be suitable for topical administration.

In further embodiments, the CD40:CD154 binding interruptor is provided indirectly to the recipient, by administration of a vector or other expressible genetic material encoding the interruptor. The genetic material is internalized and expressed in cells or tissue of the recipient, thereby producing the interruptor in situ. For example, a suitable nucleic acid construct would comprise sequence encoding one or more of the MAb 5c8 immunoglobulin (Ig) chains as disclosed in U.S. Pat. 5,474,771. Other suitable constructs would comprise sequences encoding chimeric or humanized versions of the MAb 5c8 Ig chains or antigen-binding fragments thereof. Still other suitable constructs would comprise sequences encoding part or all of other CD154-specific MAbs. The construct is delivered systemically or locally, e.g., to a site vicinal to the site of implantation of insulin-expressing tissue.

Alternatively, the vector or other genetic material encoding the interrupter is internalized within a suitable population of isolated cells to produce interuptor-producing host cells. These host cells then are implanted or infused into the recipient, either locally or systemically, to provide in situ production of the CD40:CD 154 binding interruptor. Appropriate host cells include cultured cells, such as immortalized cells, as well as cells obtained from the recipient (e.g., peripheral blood or lymph node cells, such as natural killer (NK) cells).

### Formulation

In general, the compound(s) used in practice of the invention are suspended, dissolved or dispersed in a pharmaceutically acceptable carrier or excipient. The resulting therapeutic composition does not adversely affect the recipient's homeostasis, particularly electrolyte balance. Thus, an exemplary carrier comprises normal physiologic saline (0.15M NaCl, pH 7.0 to 7.4). Another exemplary carrier comprises 50 mM sodium phosphate, 100 mM sodium chloride. Many other acceptable carriers are well known in the art and are described, for example, in Remington's Pharmaceutical Sciences, Gennaro, ed., Mack Publishing Co., 1990. Acceptable carriers can include biocompatible, inert or bioabsorbable salts, buffering agents, oligo- or polysaccharides, polymers, viscosity-improving agents, preservatives, and the like.

Any CD40:CD154 binding interruptor, such as a CD154 blocking agent, that is used in practice of the invention is formulated to deliver a pharmaceutically-effective or therapeutically-effective amount or dose, which is an amount sufficient to produce a detectable, preferably medically beneficial effect on the recipient. Medically beneficial effects would include preventing, delaying or attenuating deterioration of, or detectably improving, the recipient's medical condition. As an example, the titer of an inhibitory antibody specific for a needed, exogenous protein therapeutic can be suppressed or lowered. Thus, for example, an effective amount of a therapeutic compound of the invention, such as a CD 154 blocking agent, is any amount which detectably restores therapeutic efficacy of the protein therapeutic. An optimal effective amount is one which substantially frees the subject of counter-adaptive antibodies that give rise to the inhibitor syndrome.

### Dosages and Frequency of Treatment

The amount of and frequency of dosing for any particular compound to be used in practice of the invention is within the skills and clinical judgement of ordinary practitioners of the medical arts, such as physicians. The general dosage and administration regime is established by preclinical and clinical trials, which involve extensive but routine studies to determine effective, e.g., optimal, administration parameters for the desired compound. Even after such recommendations are made, the practitioner will often vary these dosages for different subjects based on a variety of considerations, such as the subject's age, medical status, weight, sex, and concurrent treatment with other pharmaceuticals. Determining effective dosage and administration regime for each CD40:CD154 binding interruptor used in the invention is a routine matter for those of skill in the pharmaceutical and medical arts. The dosage amount and timecourse of should be sufficient to produce a clinically beneficial change in one or more indicia of the subject's health status. Exemplary timecourse and dosage regimes are set forth in the proof-of-principle studies included herein.

To exemplify dosing considerations for an anti-CD 154 compound, the following examples of administration strategies are given for an anti-CD154 MAb. The dosing amounts could easily be adjusted for other types of CD154 blocker compounds. In general, single dosages of between about 0.05 and about 50 mg/kg subject body weight are contemplated, with dosages most frequently in the 1-20 mg/kg range. To initiate CD 154 blockade therapy prophylactically, when the subject is in remission, or for emergency therapy of acute disease, an effective dose of MAb ranges from about 1 mg/kg body weight to about 20 mg/kg body weight, administered daily or at intervals ranging from two to five days, for a period of about three weeks. Therapy can be maintained by administering the MAb intermittently thereafter, in dosages ranging from about 0.1 mg/kg body weight to about 20 mg/kg body weight. For maintenance purposes, the interdose interval may range from about one week up to about three months. At present, a one-month (four week) interdose interval is preferred.

CD154 blockade therapy can be practiced, if desired, serially or in combination with conventional immunosuppression therapy. A conventional immunosuppressant agent (e.g., a corticosteroid or calcineurin inhibitor) can be co-administered at any point during CD 154 blockade therapy deemed prudent by the practitioner. Alternatively, a CD 154 blocking MAb may be conjugated to a conventional agent. This advantageously permits the administration of the conventional agent in an amount less than the conventional dosage, for example, less than about 50% of the conventional dosage, when the agent is administered as monotherapy. Accordingly, the occurrence of many side effects associated with that agent should be avoided. Thus, according to this invention, CD154 blocking MAbs can be used together with other agents targeted at B cells, such as anti-CD19, anti-CD28 or anti-CD20 antibody (unconjugated or radiolabeled), IL-14 antagonists, LJP394 (LaJolla Pharmaceuticals receptor blocker), IR-1116 (Takeda small molecule) and anti-Ig idiotype monoclonal antibodies. Alternatively, the combinations may include T cell/B cell targeted agents, such as CTLA4Ig, IL-2 antagonists, IL-4 antagonists, IL-6 antagonists, receptor antagonists, anti-CD80/CD86 monoclonal antibodies, TNF, LFA1/ICAM antagonists, VLA4/VCAM antagonists, brequinar and IL-2 toxin conjugates (e.g., DAB), prednisone, anti-CD3 MAb (OKT3), mycophenolate mofetil (MMF), cyclophosphamide, and other immunosuppressants such as calcineurin signal blockers, including without limitation, tacrolimus (FK506). Combinations may also include T cell targeted agents, such as CD4 antagonists, CD2 antagonists and IL-12.

### Pre-Clinical Model Systems for Evaluating CD40:CD154 Interruptor Treatment Regimes

Currently preferred, exemplary model systems for testing efficacy of a CD40:CD154 interrupting compound (e.g., an anti-CD40L compound or a CD154 blocking agent, such as a MAb having the specificity of MAb 5c8) are set forth below. In each system, routine modifications or adaptations can be made, to tailor the published techniques as needed to assess the effects of any desired CD40:CD154 interrupting compound on the status of protein inhibitory titers in the model animal. Some exemplary modifications are mentioned in the following brief summaries; however, many other appropriate modifications will be apparent to the skilled practitioner and are contempleted herein.

### Knockout Mouse Model for Hemophilia A.

Recently, investigators at the American Red Cross have established a breeding colony of mice rendered nullizygous ("knocked out") for native murine FVIII. Bi et al. (1995), 10 Nature Genetics 119. These mice exhibit all relevant pathologies of human hemophilia A. Furthermore, the mouse model accurately mimics the etiology of these disease pathologies: hereditary or congenital absence of biologically active, native FVIII. Bolus administration of human FVIII, administered in a manner corresponding to conventional FVIII replacement therapy, has been reported to trigger the production of FVIII inhibitor antibodies in these mice. Quian et al. (1996), 88 Blood 656a (suppl.). Other routes of administration, specifically constitutive replacement via integration of an adenoviral vector encoding functional FVIII, appear currently to present the protein therapeutic in a less immunogenic context. Connely et al. (1998), 91 Blood 3273-3281.

The effects of a CD 154 blocking agent, e.g., an anti-murine CD 154 on the development of "high responders" in a population of the above-described hemophiliac mice can be assessed generally as follows: the antigen (FVIII) can be injected as a bolus dose (e.g., 0.2 µg) on study days 0 and 14. On or about study day 54, a blood sample can be withdrawn and assayed (using routine ELISA techniques) for presence of FVIII inhibitory antibodies. Thereafter, a test group (e.g., 5 or more animals; a similar number of animals can be assigned to one or more appropriate control groups) can be provided with an appropriate dose of the anti-murine CD 154 (e.g., 250 µg, i.p. or i.v.), for example on or about study days 55 and/or 57. A challenge dose of FVIII can be administered on or about study day 56. Thereafter, blood samples can be withdrawn and assayed on appropriate study days to monitor the development and, in the test group, suppression or reversal of a secondary response of inhibitor antibodies to FVIII. For example, bloods can be obtained at or about study days 74, 81 and 96. Allowing for some individual variation between animals in the test group, it is expected that CD154 blockade therapy will significantly blunt or suppress secondary humoral immunity to FVIII.

### SCID-hu Chimeric Mouse Model.

This chimeric mouse model system, originally reported by Mosier et al. (1988), 335 Nature 256-259, is based on immunological rescue (functional reconstitution) of severe combined immunodeficiency (SCID) mice by engraftment of normal human peripheral blood leukocytes (PBLs), resulting in a stable mouse-human chimera. This system has been used for numerous investigations of the behavior and dynamic interactions of human lymphocytes in vivo. Significantly, this model system has been used to investigate the effects of CD40:CD154 interrupting agents on the response of normal human leukocytes to murine erythrocytes (used as a model antigen). Chen et al. (1995), 155 J. Immunol. 2833-2840. In this study, anti-CD40 and anti-CD 154 MAbs were shown to downmodulate total human Ig production.

This model system allows assessment of the effects of an anti-human CD154, e.g., hu5c8, on human T cells in vivo, using any desired protein as a test antigen. In one appropriate modification, SCID-hu mouse chimeras can be created by engraftment of human PBLs from hemophiliac subjects, such as high responders. Of course, this approach can be taken with PBLs from any human affected by an exogenous protein inhibitor syndrome. In the case of SCID-hu mice made from a hemophiliac high responder, appropriate numbers (e.g., 2 to 5 or more) mice can be assigned to study groups as follows: Group A (hu5c8 and FVIII); Group B (hu5c8 alone); Group C (FVIII alone); Group D (vehicle only); Group E (control Ig and FVIII); Group F (control Ig alone). The indicated test article and/or control is admixed with the hu PBLs at the time of engraftment, and is provided i.p. on or about study days 2 and/or 4. Kinetics of the ensuing FVIII inhibitor response can be monitored by standard techniques (ELISA) using bloods withdrawn at suitable intervals over a several week period. Treatment with hu5c8 is expected to blunt or abrogate secondary humoral immunity to FVIII.

### Non-human Primate Models.

AVONEX (IFN β) model. Rhesus or cynomologus monkeys are assigned to appropriate study groups, e.g., two to four animals per group, as follow: Group 1 (control antigen (HAS); 50 µg/kg and hu5c8, 5; mg/kg), Group 2 (vehicle and hu5c8; 5 mg/kg), Group 3 (AVONEX; 50 µg/kg and hu5c8; 5 mg/kg), Group 4 (AVONEX; 50 µg/kg and hu5c8; 5 mg/kg), Group 5 (vehicle and hu5c8; 5 mg/kg). Groups 1, 2 and 3 receive hu5c8 commencing on study day 1 and approximately every second or third day thereafter. Groups 4 and 5 receive hu5c8 commencing on study day 17 and approximately every second or third day thereafter. All AVONEX groups receive AVONEX q.o.d. beginning on or about study day 3. The development and kinetics of AVONEX inhibitor antibodies are monitored using routine ELISA techniques. Clear differences are expected between the AVONEX groups treated or untreated with hu5c8. Specifically, pretreatment with hu5c8 is expected to substantially blunt or abrogate the development of AVONEX inhibitor antibodies. Delayed treatment with hu5c8 is expected to substantially suppress or reverse the development of secondary humoral immunity to AVONEX.

The above-described model can be routinely adapted to assess the effects of hu5c8 or another CD 154 blocking agent on primary and/or secondary inhibitor responses to other model antigens, including exogenous protein therapeutics. For example, routine, appropriate modifications of the protocol and dose levels can be made to assess the behavior of FVIII or another clotting factor in primates provided with prophylactic or therapeutic regimens of CD154 blockade therapy.

## Claims

1. Use of a CD40:CD154 binding interruptor in the manufacture of a medicament for attenuating severity of exogenous protein inhibitor syndrome in a subject afflicted with, or at risk of, said syndrome;
wherein the CD40:CD154 binding interruptor is a monoclonal antibody having the antigen-specific binding characteristics of the 5c8 antibody produced by ATCC Accession No. HB 10916;
wherein the exogenous protein is Factor VIII or Factor IX;
wherein the subject is human; and
wherein the human is a hemophiliac.

## Patentansprüche

1. Verwendung eines Mittels, das die Bindung CD40:CD154 hemmt, zur Herstellung eines Arzneimittels zur Verringerung des Schweregrades des exogenen Protein-Inhibitor-Syndroms in einem Subjekt, das von dem Syndrom betroffen ist oder ein Risiko dafür aufweist;
wobei das Mittel, das die CD40:CD154 Bindung hemmt, ein monoklonaler Antikörper mit den Antigen-spezifischen Bindungseigenschaften des 5c8 Antikörpers ist, der durch die unter der Zugriffsnr. ATCC HB 10916 hinterlegten Zellen produziert wird;
wobei das exogene Protein der Faktor VIII oder Faktor IX ist;
wobei das Subjekt ein Mensch ist; und
wobei der Mensch ein Hämophiliepatient ist.

## Revendications

1. Utilisation d'un interrupteur de la liaison CD40: CD154 dans la fabrication d'un médicament pour atténuer la gravité du syndrome inhibiteur de la protéine exogène chez un sujet affligé ou sous le risque dudit syndrome;
où l'interrupteur de la liaison CD40:CD154 est un anticorps monoclonal ayant les caractéristiques spécifiques de liaison de l'antigène de l'anticorps 5c8 produit par le n° d'accession ATCC HB10916;
où la protéine exogène est le Facteur VIII ou le Facteur IX;
où le sujet est humain et
où l'humain est hémophile.
